# EUROPÄISCHE PATENTSCHRIFT

(11) **EP 1 828 084 B1**
(45) Veröffentlichungstag und Bekanntmachung des Hinweises auf die Patenterteilung: **18.06.2008**
(21) Anmeldenummer: 05850281.6
(22) Anmeldetag: 15.12.2005
(51) Int. Cl.: C07C 17/02, C07C 19/045

(54) **VERFAHREN ZUR HERSTELLUNG VON 1,2-DICHLORETHAN MITTELS DIREKTCHLORIERUNG**
METHOD FOR PRODUCING 1,2-DICHLOROETHANE BY MEANS OF DIRECT CHLORINATION
PROCEDE DE FABRICATION DE 1,2-DICHLORETHANE PAR CHLORATION DIRECTE

(30) Priorität: 22.12.2004 DE 102004063090
(43) Veröffentlichungstag der Anmeldung: 05.09.2007
(73) Patentinhaber: Uhde GmbH, 44141 Dortmund (DE)
(72) Erfinder: HAFENSCHER, Harald, 65843 Sulzbach (DE); WEIS, Reinhold, 65779 Kelkheim (DE); BENJE, Michael, 64289 Darmstadt (DE)
(86) Internationale Anmeldenummer: PCT/EP2005/013535
(87) Internationale Veröffentlichungsnummer: WO 2006/069640

(56) Entgegenhaltungen:
- WO-A-03/070673
- DE-A1- 19 910 964

## Beschreibung

Die Erfindung richtet sich auf ein Verfahren zur Herstellung von 1,2-Dichlorethan, im folgenden als EDC bezeichnet, welches überwiegend als Zwischenprodukt der Herstellung von monomerem Vinylchlorid, im folgenden als VCM bezeichnet, dient, woraus letztlich Polyvinylchlorid, PVC, hergestellt wird. Bei der Umsetzung von EDC zu VCM entsteht Chlorwasserstoff HCl. EDC wird daher bevorzugt aus Ethylen C₂H₄ und Chlor Cl₂ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

Cl₂ + C₂H₄ → C₂H₄Cl₂ (Rein-EDC) + 180 kJ/Mol (1)

C₂H₄Cl₂ (Spalt-EDC) → C₂H₃Cl (VCM) + HCl - 71 kJ/Mol (2)

C₂H₄ + 2 HCl + ½ O₂ → C₂H₄Cl₂ (Roh-EDC) + H₂O + 238 kJ/Mol (3)

Das Verfahren zur Herstellung von VCM mit ausgewogener HCl-Bilanz, im folgenden kurz "ausgewogenes VCM-Verfahren" genannt, besitzt:
- eine Direktchlorierung, in der aus Ethylen C₂H₄ und Chlor Cl₂ der eine Teil des benötigten EDC in Gegenwart eines Homogenkatalysators erzeugt wird und als sogenanntes Rein-EDC abgegeben wird;
- eine Oxichlorierung, in der aus Ethylen C₂H₄, Chlorwasserstoff HCl und Sauerstoff O₂ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC und optional zusammen mit dem Rein-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen, wahlweise kann auch das aus der Direktchlorierung stammende Rein-EDC in der Hochsiederkolonne der EDC-Destillation mitdestilliert werden;
- eine EDC-Pyrolyse, in der das Feed-EDC thermisch gespalten wird; das Spaltgas genannte Reaktoraustrittsgemisch enthält VCM, Chlorwasserstoff HCl und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Als Reaktionsmedium in der Direktchlorierung dient bei den meisten im industriellen Maßstab angewandten Verfahren ein umlaufender Strom des Reaktionsprodukts EDC. Dieser kann in einem Schlaufenreaktor mit äußerem oder innerem Umlauf erzeugt werden. Weiterhin kann der Umlaufstrom durch Zwangsumlauf oder Naturumlauf erzeugt werden. Als Katalysator wird vor allem Eisen-III-chlorid verwendet; zusätzlich kann Natriumchlorid, das in der Lage ist, die Bildung von Hochsiedern zu vermindern, als Additiv verwendet werden.

Der Stand der Technik zur Direktchlorierung wird z.B. in der DE 199 10 964 A1 beschrieben. In dem in DE 199 10 964 A1 beschriebenen Verfahren sollen Nebenreaktionen, vor allem die Weiterchlorierung des EDC zum 1,1,2-Trichlorethan, dadurch unterdrückt werden, dass die Chlorierungsreaktion weitgehend in homogener, flüssiger Phase abläuft. Das in EDC schwerer als Chlor lösliche Ethylen wird im Hauptstrom des umlaufenden Reaktionsmediums EDC in einer Gleichstromblasensäule vollständig aufgelöst. Das in EDC leichter als Ethylen lösliche Chlor wird in einem unterkühlten EDC-Teilstrom aufgelöst und die so erhaltene Lösung von Chlor in EDC dem umlaufenden Hauptstrom, in dem das Ethylen bereits gelöst vorliegt, zugegeben.

Die Reaktion (1) wird üblicherweise mit einem geringen Ethylenüberschuss betrieben, um Korrosionsprobleme im Reaktionssystem, Nebenproduktbildung nach Abschluss der Direktchlorierungsreaktion im sowie andere Probleme, die mit der Behandlung chlorhaltiger Abgasströme verbunden sind, auf jeden Fall zu vermeiden. Chlor und Ethylen werden dem Reaktor über eine Verhältnisregelung zugeführt; als Führungsgrösse dient der Ethylengehalt des Reaktoraustrittssstroms. Hierbei ist man stets bestrebt, den Ethylenüberschuss am Reaktoraustritt so niedrig wie möglich zu halten, um zu grosse Ethylenverluste zu vermeiden.

Es hat sich ferner herausgestellt, dass die Reaktion (1) besonders dann ohne große Nebenproduktbildung abläuft, wenn sie vollständig als Flüssigphasenreaktion betrieben wird, wie es auch in der WO 03/070673 A1 beschrieben wird. Hierzu ist es erforderlich, dass das Ethylen im Reaktionsrohr noch vor der Zugabe von gelöstem Chlor vollständig aufgelöst ist. Die vom Gasverteiler anfänglich erzeugten kleinen Gasblasen wachsen längs dieser Strecke durch Kolaeszenz an und erreichen schliesslich eine durch Kolaeszenz - und Zerfallsvorgänge bedingte, konstante Gleichgewichtsgröße. Hier handelt es sich um einen Effekt, der den Stoffaustausch ungünstig beeinflusst, da sich durch die Vergrösserung des Blasendurchmesser bei einem bestimmten Gasgesamtvolumen die für den Stoffaustausch zu Verfügung stehende Oberfläche verkleinert.

Die Reaktion (1) in der anschließenden, weitgehend homogenen Reaktionszone verläuft kinetisch nach einem Geschwindigkeitsgesetz 2. Ordnung, also zunächst sehr schnell. Gegen Ende der Reaktionszone, wenn die Konzentrationen von Ethylen und Chlor klein werden, sinkt die Reaktionsgeschwindigkeit stark ab.

Die Überlagerung der Effekte beim Lösungsverhalten des Ethylen, bei der Reaktion selbst und dem Einsetzen des Siedens definieren beim herkömmlichen Stand der Technik die Dimensionierung des Siedereaktors und erschweren eine nachträgliche Kapazitätserhöhung erheblich.

Die Aufgabe der Erfindung besteht daher darin, ein wirtschaftliches Verfahren zur Verfügung zu stellen, welches auf kleinem Raum eine hohe Raumausbeute an Produkt ermöglicht und somit eine Kapazitätsvergrößerung ermöglicht, ohne die äußeren Reaktorabmessungen zu vergrößern, und welche gleichzeitig EDC hoher Reinheit erzeugt.

Diese Aufgabe wird durch das Verfahren nach Patentanspruch 1 gelöst. Die Erfindung löst die Aufgabe, wobei die Zugabestellen von Chlor und gelöstem Ethylen in dem Schenkel der Schlaufe angeordnet sind, in dem die Flüssigkeit aufsteigt, wobei stets einer stromaufwärts gelegenen Zugabestelle von Ethylen eine stromabwärts gelegene Zugabestelle von gelöstem Chlor folgt, indem
- jeder Zugabestelle von Chlor mindestens eine Zugabestelle von EDC folgt, und
- die Zugabe des flüssigen EDC unter so hoher kinetischer Energie erfolgt, dass eine starke Vermischung von EDC, gelöstem Chlor und Ethylen erfolgt.

Flüssiges EDC steht in den meisten Anlagen ohnehin zur Verfügung, da es üblicherweise aus dem Reaktionsgefäß abgezogen und der Wärmerückgewinnung zugeführt wird. Das leicht abgekühlte EDC wird üblicherweise in den Schenkel des Reaktionsgefäßes zurückgeführt, in dem die Flüssigkeit absinkt. Hierdurch kann der absinkenden Flüssigkeit ein zusätzlicher Impuls verliehen werden, der den Naturlauf verstärkt. Es wurde jetzt gefunden, dass es dieser Impuls-Unterstützung nicht bedarf, wenn die Raumausbeute, und damit verbunden, der Gesamtumsatz an EDC entsprechend vergrößert wird, weil der hierdurch erreichte thermische Effekt ebenfalls zu einer entsprechenden Verstärkung des Naturumlaufs führt. Innerhalb der Zone, in der die Hauptreaktion stattfindet, steht auch weiterhin keine Gas-Flüssig-Phasengrenzfläche zur Verfügung, welche die Bildung von Nebenprodukten, besonders von 1,1,2 - Trichlorethan katalysieren könnte.

In einer weiteren Ausgestaltung der Erfindung wird vorgesehen, dass die Zugabe des flüssigen 1,2-Dichlorethans mittels eines oder mehreren Strahlmischern, welche auch als Tankmischer bezeichnet werden, erfolgt. Die Betriebsweise eines solchen Mischers entspricht der einer Flüssigkeitsstrahl-Flüssigkeitspumpe. In typischen Anwendungen wird der Strahlmischer zur Durchmischung des Inhalts von Flüssigkeitsbehältern oder -Tanks verwendet, um die Ausbildung von Temperatur- oder Konzentrationsgradienten zu unterdrücken. Der Mischer wird untergetaucht betrieben; mittels der kinetischen Energie des Treibstrahls wird umgebendes Medium angesaugt und sowohl mit dem Treibmedium als auch mit dem umgebenden Behälterinhalt vermischt. Der Austrittsstrom des Strahlmischers beträgt ein mehrfaches des Treibstrahls, so dass auch grosse Behältervolumina in kurzer Zeit durchmischt werden können. Die vorliegende Erfindung zielt darauf ab, durch Verwendung von Strahlmischern die kinetische Energie des im Kreislauf geführten EDC auszunutzen, um die Reaktionspartner Chlor und Ethylen stromabwärts der Zugabestelle von gelöstem Chlor möglichst schnell zu vermischen.

Weitere Ausgestaltungen der Erfindung betreffen die Anordnung des Strahlmischers oder, wenn mehrere Strahlmischer eingesetzt werden, der Strahlmischer. Der oder die Strahlmischer können innerhalb des Schlaufenreaktors so angeordnet sein, dass die sie verlassende Flüssigkeit entweder eine, im Rohrquerschnitt gesehen, tangentiale Strömung erzeugt, die der aufwärts gerichteten Hauptströmungsrichtung überlagert ist, oder auch eine, im Längsschnitt gesehen, aufwärts gerichtete Strömung erzeugt, die die Aufwärtsströmung verstärkt, oder so, dass beide Strömungsrichtungen unterstützt werden. Die Strahlmischer-Anordnung erscheint sowohl im Längsschnitt als auch im Rohrquerschnitt als quer zur Hauptströmungsrichtung gelegen.

Im letzteren Fall ist die Strömungsaustrittsrichtung aus dem Strahlmischer oder den Strahlmischern schräg nach oben geneigt. Bei der Ausrichtung ist es unschädlich, wenn der Strömungsaustritt auch eine radiale Komponente enthält, hierdurch wird das Mischungsverhalten nicht wesentlich beeinträchtigt, jedoch auch nicht gefördert. Eine nach oben geneigte Ausrichtung wird der Fachmann besonders dann wählen, wenn in einer weiteren Ausgestaltung der Erfindung oberhalb der Strahlmischer-Ebene noch ein statischer Mischer angeordnet wird.

Insgesamt ist es auf diese Weise möglich, mit einem Siedereaktor herkömmlicher Baugröße durch Umbau in der erfindungsgemäßen Weise den doppelten Umsatz nach Reaktion (1) zu erreichen. Der große Vorteil der Erfindung besteht daher in der unkomplizierten Nachrüstbarkeit bei Kapazitätserhöhungen in bestehenden Anlagen. Selbstverständlich ist es auch wirtschaftlich besonders bei großen Anlagen sinnvoll, Siedereaktoren schon bei der ursprünglichen Planung mit den erfindungsgemäßen Aufgabevorrichtungen auszustatten.

Die Erfindung umfasst auch die Vorrichtung zur Durchführung des Verfahrens mit einem Siedereaktor, welcher aus einem Ausgasgefäß, einer im Naturumlauf betriebenen Reaktionsschlaufe sowie Abzugsvorrichtungen für erzeugtes EDC besteht, und mindestens in einer Ebene einen oder mehrere Strahlmischer, die wie oben beschrieben angeordnet sind, aufweist. Die Vorrichtung kann optional auch statische Mischvorrichtungen enthalten.

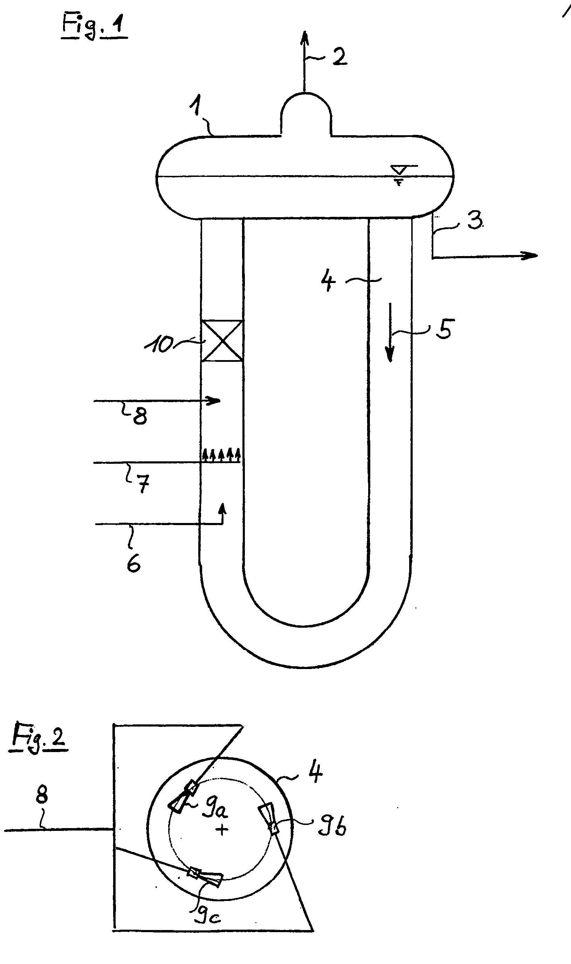

Die Erfindung wird im folgenden anhand eines Beispiels in Fig. 1 bis Fig. 3 näher erläutert. Fig. 1 zeigt einen Direktchlorierungsreaktor, der aus einem Ausgasgefäß 1, aus welchem sowohl gasförmiges EDC 2 als auch flüssiges EDC 3 abgezogen werden, und einer Schlaufe 4 besteht, in welcher flüssiges EDC 5, angedeutet durch einen Pfeil, umläuft, und in welcher die Reaktion (1) durchgeführt wird. Im aufsteigenden Teil der Schlaufe 4 befinden sich nacheinander die Zugabestellen für Ethylen 6, gelöstem Chlor 7 und EDC 8, wobei sich jedoch auch eine Vielzahl solcher Zugabestellen im Schlaufenreaktor befinden können.

Fig. 2 zeigt in einem Rohrquerschitt durch den aufsteigenden Teil der Schlaufe 4 die Anordnung von in diesem Beispiel 3 Strahlmischern 9a, 9b und 9c, die mit EDC 8 gespeist werden. Das hierbei verwendete EDC kann sowohl aus dem abgezogenen, flüssigen EDC 3 gebildet werden, nachdem dieses Wärme abgegeben hat, als auch durch kondensiertes, zuvor gasförmiges EDC 2, welches zurückgeführt wird. Selbstverständlich kommt auch eine Mischung daraus in Frage. Wie in Fig. 1 gezeigt, kann oberhalb der Strahlmischer-Ebene noch ein statischer Mischer angebracht werden.

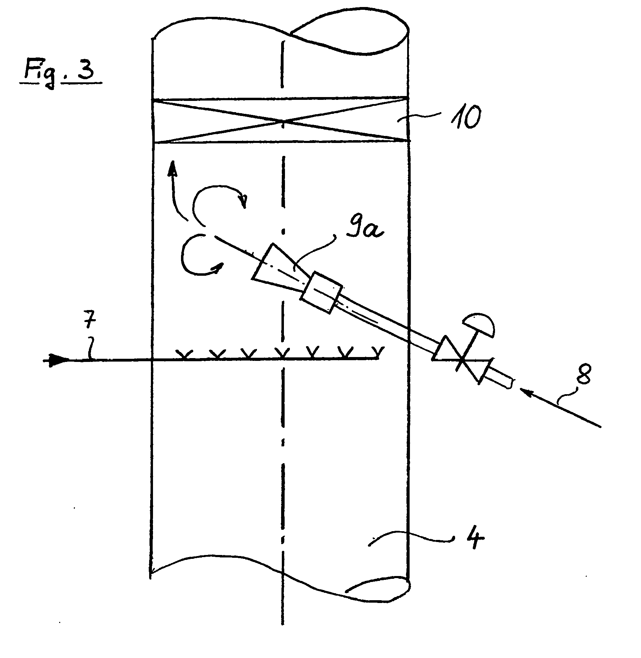

Fig.3 zeigt einen Längsschnitt durch einen aufsteigenden Teil der Schlaufe 4, in dem bereits die Lösung von Ethylen stattgefunden hat. Hierbei wird das gelöste Chlor 7 durch eine Vielzahl von Düsen über den Querschnitt verteilt eindosiert. Direkt oberhalb dieser Chlor-Eindüsung befindet sich ein durch EDC 8 gespeister Strahlmischer 9a - weitere Strahlmischer werden hier nicht gezeigt, können aber vorhanden sein - sowie oberhalb des Strahlmischers 9a ein statischer Mischer 10. Der Strahlmischer 9a ist nach oben hin ausgerichtet und unterstützt die Strömung durch Einbringung einer Impulskomponente, die etwa dem Druckverlust entsprechen sollte, die der statische Mischer verursacht, wobei gleichzeitig eine möglichst große Verwirbelung erzeugt wird.

**Bezugszeichenliste**
- 1: Ausgasgefäß
- 2: gasförmiges EDC
- 3: flüssiges EDC
- 4: Schlaufe
- 5: flüssiges EDC
- 6: Ethylen-Zugabestelle
- 7: Chlor-Zugabestelle
- 8: EDC
- 9a: Strahlmischer
- 9b: Strahlmischer
- 9c: Strahlmischer
- 10: statischer Mischer

## Patentansprüche

1. Verfahren zur Herstellung von 1,2-Dichlorethan hoher Reinheit aus gelöstem Chlor und gelöstem Ethylen, welche miteinander in Kontakt gebracht werden, unter Einsatz eines im Umlauf geführten flüssigen Reaktionsmediums, welches im Wesentlichen aus 1,2-Dichlorethan und einem Katalysator besteht und mindestens eine vertikal angeordnete, als Schlaufe ausgebildete Reaktionsstrecke durchläuft, wobei die beiden Schenkel der Schlaufe mit einem oberseitig angeordneten Ausgasbehälter verbunden sind, von dem aus das Reaktionsprodukt entweder gasförmig oder flüssig oder sowohl gasförmig als auch flüssig ausgeschleust wird, und wobei die Zugabestellen von Chlor und gelöstem Ethylen in dem Schenkel der Schlaufe angeordnet sind, in dem die Flüssigkeit aufsteigt, wobei stets einer stromaufwärts gelegenen Zugabestelle von Ethylen eine stromabwärts gelegene Zugabestellen von gelöstem Chlor folgt,
**dadurch gekennzeichnet, dass**
• jeder Zugabestelle von Chlor mindestens eine Zugabestelle von flüssigem 1,2-Dichlorethan folgt, und
• die Zugabe des flüssigen 1,2-Dichlorethans unter so hoher kinetischer Energie erfolgt, dass eine starke Vermischung von 1,2-Dichlorethan, gelöstem Chlor und Ethylen erfolgt.

2. Verfahren nach Anspruch 1, **dadurch gekennzeichnet, dass** die Zugabe des flüssigen 1,2-Dichlorethans mittels eines oder mehreren Strahlmischern erfolgt.

3. Verfahren nach Anspruch 2, **dadurch gekennzeichnet, dass** der oder die Strahlmischer innerhalb des Schlaufenreaktors so angeordnet sind, dass die sie verlassende Flüssigkeit quer zur aufwärts gerichteten Hauptströmungsrichtung gerichtet ist.

4. Verfahren nach Anspruch 2, **dadurch gekennzeichnet, dass** der oder die Strahlmischer innerhalb des Schlaufenreaktors so angeordnet sind, dass die sie verlassende Flüssigkeit eine, im Rohrquerschnitt gesehen, tangentiale Strömung erzeugt, die der aufwärts gerichteten Hauptströmungsrichtung überlagert ist.

5. Verfahren nach Anspruch 2, **dadurch gekennzeichnet, dass** der oder die Strahlmischer innerhalb des Schlaufenreaktors so angeordnet sind, dass die sie verlassende Flüssigkeit eine, im Längsschnitt gesehen, aufwärts gerichtete Strömung erzeugt, die die aufwärts gerichtete Hauptströmungsrichtung verstärkt.

6. Verfahren nach Anspruch 2, **dadurch gekennzeichnet, dass** der oder die Strahlmischer innerhalb des Schlaufenreaktors so angeordnet sind, dass die sie verlassende Flüssigkeit sowohl eine, im Rohrquerschnitt gesehen, tangentiale Strömung erzeugt, die der aufwärts gerichteten Hauptströmungsrichtung überlagert ist, als auch eine, im Längsschnitt gesehen, aufwärts gerichtete Strömung erzeugt, die die Aufwärtsströmung verstärkt.

7. Verfahren nach einem der vorangegangen Ansprüche 2 bis 6, **dadurch gekennzeichnet, dass** außer den Strahlmischern auch statische Mischer in der aufwärts gerichteten Schlaufe des Schlaufenreaktors angeordnet sind.

8. Vorrichtung zur Herstellung von 1,2-Dichlorethan hoher Reinheit aus gelöstem Chlor und gelöstem Ethylen, welche miteinander in Kontakt gebracht werden, unter Einsatz eines im Umlauf geführten flüssigen Reaktionsmediums, welches im Wesentlichen aus 1,2-Dichlorethan und einem Katalysator besteht, enthaltend
• mindestens eine vertikal angeordnete, als Schlaufe ausgebildete Reaktionsstrecke,
• wobei die beiden Schenkel der Schlaufe mit einem oberseitig angeordneten Ausgasbehälter verbunden sind, von dem aus das Reaktionsprodukt entweder gasförmig oder flüssig oder sowohl gasförmig als auch flüssig ausgeschleust wird,
• Zugabestellen für Chlor und gelöstem Ethylen in dem Schenkel der Schlaufe angeordnet sind, in dem die Flüssigkeit aufsteigt,
• wobei stets einer stromaufwärts gelegenen Zugabestelle von Ethylen eine stromabwärts gelegene Zugabestelle von gelöstem Chlor folgt,
**dadurch gekennzeichnet, dass**
• jeder Zugabestelle von Chlor mindestens eine Zugabestelle von flüssigem 1,2-Dichlorethan folgend angeordnet ist, und
• zur Zugabe des flüssigen 1,2-Dichlorethans eine Vorrichtung angebracht ist, die geeignet ist, unter Zuführung hoher kinetischer Energie eine starke Vermischung von 1,2-Dichlorethan, gelöstem Chlor und Ethylen zu bewirken.

9. Vorrichtung nach Anspruch 8, **dadurch gekennzeichnet,dass** die Vorrichtung, die geeignet ist, unter Zuführung hoher kinetischer Energie eine starke Vermischung von 1,2-Dichlorethan, gelöstem Chlor und Ethylen zu bewirken, aus einem oder mehreren Strahlmischern besteht.

10. Vorrichtung nach Anspruch 9, **dadurch gekennzeichnet, dass** der oder die Strahlmischer innerhalb des Schlaufenreaktors so angeordnet sind, dass die sie verlassende Flüssigkeit quer zur aufwärts gerichteten Hauptströmungsrichtung gerichtet ist.

11. Vorrichtung nach Anspruch 9, **dadurch gekennzeichnet, dass** der oder die Strahlmischer innerhalb des Schlaufenreaktors so angeordnet sind, dass die sie verlassende Flüssigkeit eine, im Rohrquerschnitt gesehen, tangentiale Strömung erzeugt, die der aufwärts gerichteten Hauptströmungsrichtung überlagert ist.

12. Vorrichtung nach Anspruch 9, **dadurch gekennzeichnet, dass** der oder die Strahlmischer innerhalb des Schlaufenreaktors so angeordnet sind, dass die sie verlassende Flüssigkeit eine, im Längsschnitt gesehen, aufwärts gerichtete Strömung erzeugt, die die aufwärts gerichtete Hauptströmungsrichtung verstärkt.

13. Vorrichtung nach Anspruch 9, **dadurch gekennzeichnet, dass** der oder die Strahlmischer innerhalb des Schlaufenreaktors so angeordnet sind, dass die sie verlassende. Flüssigkeit sowohl eine, im Rohrquerschnitt gesehen, tangentiale Strömung erzeugt, die der aufwärts gerichteten Hauptströmungsrichtung überlagert ist, als auch eine, im Längsschnitt gesehen, aufwärts gerichtete Strömung erzeugt, die die Aufwärtsströmung verstärkt.

14. Vorrichtung nach einem der vorangegangen Ansprüche 9 bis 13, **dadurch gekennzeichnet, dass** außer den Strahlmischern auch statische Mischer in der aufwärts gerichteten Schlaufe des Schlaufenreaktors angeordnet sind.

## Claims

1. Process for the production of 1,2 dichloroethane of high purity from dissolved chlorine and dissolved ethylene that come into contact with each other in the presence of a circulated liquid reaction agent, the said agent primarily consisting of 1,2 dichloroethane and a catalyst, using at least one reaction unit arranged vertically and designed as loop, the two leg sections of the loop communicating with a degassing vessel arranged above the unit, the degassing vessel serving for the withdrawal of the reaction product in gaseous or liquid form or in both forms, the feed points for chlorine and dissolved ethylene being located in the loop leg section in which the liquid ascends, any upstream feed point for ethylene being followed by a downstream feed point for dissolved chlorine, **characterised in that**
• any feed point for chlorine is followed by at least one feed point for liquid 1,2-dichloroethane, and
• the addition of liquid 1,2-dichloroethane takes place with so much kinetic energy that a thorough mixture of 1,2-dichloroethane, dissolved chlorine and ethylene is effected.

2. Process in accordance with Claim 1, **characterised in that** the addition of liquid 1,2- dichloroethane is effected by means of one or several jet mixer/s.

3. Process in accordance with Claim 2, **characterised in that** the jet mixer(s) is/are arranged in the loop reactor in such a manner that the liquid leaving the mixers flows crosswise to the upward main stream direction.

4. Process in accordance with Claim 2, **characterised in that** the jet mixer/s is/are arranged in the loop reactor in such a manner that the liquid leaving the mixers generates a tangential stream as seen from pipe cross section, the said stream overlapping and extending over the upward main stream direction.

5. Process in accordance with Claim 2, **characterised in that** the jet mixer/s is/are arranged in the loop reactor in such a manner that the liquid leaving the mixer/s generates an upward stream as seen from the longitudinal section, the said stream reinforcing the upward main stream direction.

6. Process in accordance with Claim 2, **characterised in that** the jet mixer/s is/are arranged in the loop reactor in such a manner that the liquid leaving the mixer/s generates an upward stream as seen from the longitudinal section, the said stream reinforcing the upward main stream direction.

7. Process in accordance with any of the preceding Claims 2 to 6, **characterised in that** it is also possible to combine a static mixer with the jet mixers, the static mixer being arranged in the upward loop section of the loop reactor.

8. Device for the production of high-purity 1,2-dichloroethane from dissolved chlorine and dissolved ethylene which come into contact with each other in the presence of a circulated liquid reaction agent, the reaction agent mainly consisting of 1,2-dichloroethane and a catalyst, using the equipment items described below:
• At least one reaction unit arranged vertically and designed as loop,
• the two legs of the loop communicating with a degassing vessel arranged above the unit, the said vessel serving for the withdrawal of the reaction product, either in gaseous or liquid form or in both forms,
• the feed points for chlorine and dissolved ethylene being located in the loop leg section in which the liquid ascends,
• any upstream feed point for ethylene being followed by a downstream feed point for dissolved chlorine,
**characterised in that**
• any feed point for chlorine is followed by at least one feed point for 1,2-dichloroethane, and
• the addition of liquid 1,2-dichloroethane takes place with so much kinetic energy that a thorough mixture of 1,2-dichloroethane, dissolved chlorine and ethylene is effected.

9. Device in accordance with Claim 8, **characterised in that** the device suitable for the feed at high kinetic energy so that a thorough mixture of 1,2-dichloroethane, dissolved chlorine and ethylene is ensured, consists of several jet mixers.

10. Device in accordance with Claim 9, **characterised in that** the jet mixer/s is/are arranged in the loop reactor in such a manner that the liquid leaving the mixer/s flows crosswise to the upward main stream direction.

11. Device in accordance with Claim 9, **characterised in that** the jet mixer/s is/are arranged in the loop reactor in such a manner that the liquid leaving the mixer/s generates a tangential stream as seen from the tube cross-section, the said stream overlapping and extending over the upward main stream direction.

12. Device in accordance with Claim 9, **characterised in that** the jet mixer/s is/are arranged in the loop reactor in such a manner that that the liquid leaving the mixer/s generates an upward stream as seen from the longitudinal section, the said stream reinforcing the upward main stream direction.

13. Device in accordance with Claim 9, **characterised in that** the jet mixer/s is/are arranged in the loop reactor in such a manner that the liquid leaving the mixer/s generates a tangential stream as seen from the tube cross-section, the said stream overlapping the upward main stream direction, as well as an upward flow as seen from the longitudinal section, the upward stream thus being reinforced.

14. Device in accordance with any of the preceding Claims 9 to 13, **characterised in that** it is also possible to combine a static mixer with the jet mixers, the static mixer being arranged in the upward loop section of the loop reactor.

## Revendications

1. Procédé pour la production de 1,2-dichloroéthane, de haute pureté, à partir de chlore dissous et d'éthylène dissous, qui sont mis au contact entr'eux, en présence d'un flux circulé d'un agent de réaction liquide, qui consiste principalement en 1,2-dichloroéthane et un liquide catalytique et qui parcourt au minimum une unité verticale de réaction dessinée comme une boucle, dont les deux sections de côté sont branchées sur un récipient de dégazage disposé au-dessus, le dit récipient de dégazage étant utilisé pour l'évacuation du produit de réaction sous forme gazeuse ou liquide ou bien sous forme gazeuse et liquide; les points d'amenée de chlore et d'éthylène dissous sont disposés dans la section de côté de la boucle dans laquelle le liquide monte vers le haut, et chaque point d'amenée d'éthylène disposé en amont est suivi, au moins, d'un point d'amenée de chlore dissous disposé en aval, **caractérisé en ce que**
• chaque point d'amenée de chlore est suivi, au moins, d'un point d'amenée de 1,2-dichloroéthane, et que
• l'addition du 1,2-dichloroéthane a lieu à l'aide d'une énergie cinétique suffisamment élevée pour assurer un mélange intense du 1,2-dichloroethane, du chlore dissous et de l'éthylène.

2. Procédé selon la revendication no. 1, **caractérisé en ce que** l'addition du 1,2-dichloroéthane a lieu à l'aide d'un mélangeur-éjecteur ou de plusieurs appareils de ce type.

3. Procédé selon la revendication no. 2, **caractérisé en ce que** le(s) dit(s) mélangeur(s)-éjecteur(s) est/sont disposé(s) de telle sorte que le liquide sortant du/des dit(s) appareil(s) s'écoule transversalement par rapport au flux principal dirigé vers le haut.

4. Procédé selon la revendication no. 2, **caractérisé en ce que** le(s) mélangeur(s)-éjecteur(s) est/sont disposé(s) de telle sorte que le liquide sortant du/des dit(s) appareil(s) produise un flux tangentiel par rapport à la coupe transversale du tuyau, le dit flux ayant une zone de recouvrement avec le flux principal dirigé vers le haut.

5. Procédé selon la revendiaction no. 2, **caractérisé en ce que** le(s) mélangeurs-éjecteurs est/sont disposé(s) dans le réacteur à boucle de telle manière que le liquide sortant du/des dit(s) appareil(s) provoque un flux dirigé vers le haut, par rapport à la coupe longitudinale, et que le flux principal également dirigé vers le haut soit ainsi renforcé.

6. Procédé selon la revendication no. 2, **caractérisé en ce que** le(s) mélangeur(s)-éjecteur(s) est/sont disposé(s) dans le réacteur à boucle de telle manière que le liquide sortant du/des dit(s) appareil(s) provoque un flux tangentiel, par rapport à la coupe transversale du tuyau, et que ce flux tangentiel ait une zone de recouvrement avec le flux principal dirigé vers le haut, par rapport à la coupe longitudinale, et que le flux dirigé vers le haut soit ainsi renforcé.

7. Procédé selon l'une des revendications nos. 2 à 6, **caractérisé en ce que** des mélangeurs statiques sont combinés avec des mélangeurs-éjecteurs et que les dits mélangeurs statiques sont disposés dans la section de boucle orientée vers le haut.

8. **Dispositif** pour la production de 1,2-dichloroéthane de haute pureté, à partir de chlore dissous et d'éthylène dissous, qui sont mis au contact entre'eux, en présence d'un flux circulé d'un agent de réaction liquide, qui consiste principalement en 1,2-dichloroéthane et un liquide catalytique, mettant en oeuvre l'équipement spécifié ci-après:
• Au moins, une unité de réaction en position verticale et dessinée comme une boucle ;
• les deux sections de côté de la boucle sont branchées sur un récipient de dégazage disposé au-dessus et utilisé pour l'évacuation du produit de réaction sous forme gazeuse ou liquide ou bien sous forme gazeuse et liquide;
• les points d'amenée de chlore et d'éthylène dissous sont disposés dans la section de côté de la boucle, dans laquelle le liquide monte vers le haut ;
• chaque point d'amenée d'éthylène disposé en amont est suivi, au moins, d'un point d'amenée de chlore dissous ;
**caractérisé en ce que**
• chaque point d'amenée de chlore est suivi, au moins, d'un point d'amenée de 1,2-dichloroéthane, et que
• l'addition du 1,2-dichloroéthane liquide a lieu à l'aide d'une énergie cinétique suffisamment élevée pour assurer un mélange intense du 1,2-dichloroéthane, du chlore dissous et de l'éthylène.

9. Dispositif selon la revendicatiion no. 8, **caractérisé en que** le dispositif qui est bien approprié à assurer un mélange intense de 1,2-dichloroéthane, de chlore dissous et d'éthylène, à l'aide de l'intervention d'une énergie cinétique élevée, consiste en un seul ou plusieurs mélangeurs-éjecteurs.

10. Dispositif selon la reventication no. 9, **caractérisé en ce que** le(s) mélangeur(s)-éjecteur(s) est/sont disposé(s) dans la boucle du réacteur de telle sorte que le liquide sortant du/des mélangeur(s) provoque un flux transversal par rapport au flux principal dirigé vers le haut.

11. Dispositif selon la revendication no. 9, **caractérisé en ce que** le(s) mélangeur(s)-éjecteur(s) est/sont disposé(s) dans la boucle du réacteur de telle sorte que le liquide sortant du/des mélangeur(s) provoque un flux tangentiel, par rapport à la coupe transversale du tuyau, et assure ainsi une zone de recouvrement avec le flux principal dirigé vers le haut.

12. Dispositif selon la revendication no. 9, **caractérisé en ce que** le(s) mélangeur(s)-éjecteur(s) est/sont disposé(s) dans la boucle du réacteur de telle sorte que le liquide sortant du/des mélangeur(s) provoque un flux dirigé vers le haut, par rapport à la coupe longitudinale du tuyau, et qui assure ainsi un renfort du flux principal dirigé vers le haut.

13. Dispositif selon la revendication no. 9, **caractérisé en ce que** le(s) mélangeur(s)-éjecteur(s) est/sont disposé(s) dans la boucle du réacteur de telle sorte que le liquide sortant du/des mélangeur(s) provoque un flux tangentiel, par rapport à la coupe transversale du tuyau, qui assure ainsi une zone de recouvrement avec le flux principal dirigé vers le haut, et que le flux dirigé vers le haut soit ainsi renforcé, par rapport à la coupe longituinale.

14. Dispositif selon les revendications nos. 9 à 13, **caractérisé en ce qu'**un mélangeur statique est combiné avec les mélangeurs-éjecteurs et que le dit mélangeur statique est disposé dans la section de boucle dirigé vers le haut.
